# EUROPEAN PATENT APPLICATION

(11) **EP 4 361 248 A1**
(43) Date of publication of application: **01.05.2024**
(21) Application number: 22828697.7
(22) Date of filing: 20.06.2022
(51) Int. Cl.: C12M 1/34, C12M 1/00, B01L 7/00

(54) **CELL THAWING MACHINE AND METHOD FOR OPERATING SAME**

(30) Priority: 25.06.2021 KR 20210082930; 27.07.2021 KR 20210098641; 05.11.2021 KR 20210151329
(71) Applicant: Amogreentech Co., Ltd., Gimpo-si, Gyeonggi-do 10014 (KR); Kangstem Biotech Co., Ltd., Seoul 06179 (KR)
(72) Inventor: HAN, Kyoung Ku, Gimpo-si, Gyeonggi-do 10014 (KR); JANG, Seon Ho, Gimpo-si, Gyeonggi-do 10014 (KR); SONG, Jae Kyung, Gimpo-si, Gyeonggi-do 10014 (KR); SEO, In Yong, Gimpo-si, Gyeonggi-do 10014 (KR); KIM, Jae Yun, Gimpo-si, Gyeonggi-do 10014 (KR); KANG, Kyung Sun, Seoul 06338 (KR); LEE, Mi Hye, Seoul 08861 (KR); HEO, Hyun Suk, Daegu 41226 (KR)
(74) Representative: Treeby, Philip David William
(86) International application number: PCT/KR2022/008719
(87) International publication number: WO 2022/270850

(57) **Abstract**

Provided is a cell thawing machine. A cell thawing machine according to an embodiment of the present invention comprises: a thawing unit that includes heating blocks including a first heating block and a second heating block forming a heating space for heating a container in which a predetermined amount of biological material including cells is stored, heaters installed in the heating blocks to provide heat to the heating blocks, and insulation blocks coupled to the heating blocks so as to surround the heaters; a driving unit that provides driving force for raising and lowering support pins so that the pins can support a lower portion of the container, and moving the first heating block and the second heating block in a straight line to the left and right; and a control unit that controls the thawing unit and the driving unit.

## Description

### [Technical Field]

The present invention relates to a cell thawing machine and an operating method thereof.

### [Background Art]

In general, various biological materials are stored at low temperature after being stored in containers such as vials. For example, plasma and tissue cells are stored at a maximum of -100 degrees Celsius, and stem cells are stored at cryogenic temperatures of up to -165 degrees Celsius by using gaseous liquid nitrogen.

These biological materials are tested at a temperature higher than the storage temperature of the aforementioned low-temperature state. Accordingly, a thawing process is required to test the biological material.

To this end, a cell thawing machine has been proposed for thawing a biological material in a low-temperature state by heating a container such as a vial in which the biological material is stored.

However, conventional cell thawing machines adopt a method of transferring heat while a heating block and a container are in contact with each other. Accordingly, the conventional cell thawing machines have a problem in that it is difficult to uniformly heat the container as a whole, and since the high-temperature heating block directly contacts the container, there is a problem in that the container is cracked or deformed by the high temperature.

In addition, the conventional cell thawing machine was a method of simply displaying whether the heating block was operating normally through an indicator such as an LED.

Accordingly, the conventional cell thawing machines have a problem in that the operating temperature of the heating block cannot be easily measured during qualification evaluation to determine whether the heating block operates correctly.

### [Disclosure]

### [Technical Problem]

The present invention has been devised in view of the above points, and an object of the present invention is to provide a cell thawing machine which is capable of uniformly heating the container as a whole while securing the heat stability of the container including the biological material during the heating process, and an operation method thereof.

In addition, another object of the present invention is to provide a cell thawing machine which is capable of increasing energy use efficiency and an operating method thereof.

Moreover, still another object of the present invention is to provide a cell thawing machine which is capable of conveniently measuring the operating temperature of a heating block during qualification evaluation.

### [Technical Solution]

In order to achieve the above-described objects, the present invention provides a cell thawing machine, including a thawing part including heating blocks which include a first heating block and a second heating block forming a heating space for heating a container in which a predetermined amount of biological material including cells is stored, heaters which are installed in the heating blocks to provide heat to the heating blocks, and heat insulating blocks which are coupled to the heating blocks so as to surround the heaters; a driving part for providing a driving force for raising and lowering support pins such that the support pins can support a lower portion of the container, and linearly moving each of the first heating block and the second heating block to the left and right directions; and a control part for controlling the thawing part and the driving part.

In addition, the present invention provides a method for operating a cell thawing machine including heating blocks which include a first heating block and a second heating block for forming a heating space for accommodating a container in which a predetermined amount of biological material including cells is stored through linear movement by a driving force of a motor, and heating the container accommodated in the heating space through heat provided from a heater; and support pins which are raised and lowered to support a lower portion of the container disposed in the heating space in connection with the linear movement of the heating block, the method including a first step of preheating the first heating block and the second heating block to a predetermined temperature by driving the heater while one surface of the first heating block and one surface of the second heating block facing each other are in contact with each other; a second step of heating the container disposed in the heating space to thaw the biological material while the first heating block and the second heating block are changed to a heating position forming the heating space; and a third step of changing the first heating block and the second heating block to a standby position by moving the same in a direction away from the heating position such that the container disposed in the heating space can be removed, wherein in the second step, the container disposed in the heating space is heated by heat transferred from the first heating block and the second heating block in a non-contact manner in a state where a lower portion of the container is supported through the support pins and the container is not in contact with the first heating block and the second heating block.

### [Advantageous Effects]

According to the present invention, the container can be uniformly heated by heating the container in a non-contact heating manner by using radiant heat, and it is possible to prevent cracks or thermal deformation of the container that may occur during heating by conductive heat in advance.

In addition, according to the present invention, since the temperature of the heating block can be maintained constant, a plurality of containers can be sequentially thawed. Through this, the present invention can improve energy consumption efficiency because it is possible to thaw a plurality of containers while reducing the reuse waiting time for thawing containers.

Furthermore, according to the present invention, when the upper cover is separated, the sensor insertion hole for qualification evaluation is exposed to the outside such that it is possible to easily perform qualification evaluation.

### [Description of Drawings]

FIG. 1 is a view showing a cell thawing machine according to one embodiment of the present invention;
FIG. 2 is a view of FIG. 1 viewed from another direction;
FIG. 3 is a view in which the housing is separated from FIG. 1;
FIG. 4 is a view showing the cell thawing machine according to the first embodiment of the present invention, showing a state in which the housing and circuit board are removed from FIG. 3;
FIG. 5 is a view of FIG. 4 viewed from another direction;
FIG. 6 is a view in which the thawing part is separated from FIG. 4;
FIG. 7 is a view in which the thawing part is extracted and separated from FIG. 6;
FIG. 8 is a view in which the driving part is extracted from FIG. 6;
FIG. 9 is an enlarged view of a part of FIG. 5, in which portions of the first heating block and the second heating block are cut in a standby position;
FIG. 10 is a view showing a part of the cell thawing machine according to the first embodiment of the present invention in a state where the heating block is disposed at a heating position;
FIG. 11 is a view of FIG. 10 viewed from above;
FIG. 12 is a view showing the arrangement relationship of sensors and heating blocks corresponding to the state of FIG. 10, showing a state in which the first heating block, the first heat insulating block and the first guide block are removed;
FIG. 13 is an operating state diagram of the cell thawing machine according to the first embodiment of the present invention, and is a partial cross-sectional view of the standby position when initially driven, as viewed from the A-A direction in FIG. 10;
FIG. 14 is an operating state diagram showing a state in which the heating block is preheated in the cell thawing machine according to the first embodiment of the present invention, and is a partial cross-sectional view as viewed from the A-A direction of FIG. 10;
FIG. 15 is an operating state diagram showing the process of introducing a container into the cell thawing machine according to the first embodiment of the present invention, and is a partial cross-sectional view as viewed from the direction A-A of FIG. 10;
FIG. 16 is an operating state diagram of the cell thawing machine according to the first embodiment of the present invention, and is a partial cross-sectional view of the state in which the container is heated in the heating position, as viewed from the A-A direction in FIG. 10;
FIG. 17 is an operating state diagram of the cell thawing machine according to the first embodiment of the present invention, and is a partial cross-sectional view of the process of removing the container from the standby position, as viewed from the A-A direction in FIG. 10;
FIG. 18 is a view showing a cell thawing machine according to the second embodiment of the present invention, showing a state in which the top cover is separated from the state in which the cover is removed in FIG. 1;
FIG. 19 is a cross-sectional view in the B-B direction of FIG. 18, in which the thawing part is extracted;
FIG. 20 is a view showing a cell thawing machine according to the second embodiment of the present invention, showing a state in which the housing and circuit board are removed from FIG. 3;
FIG. 21 is a view of FIG. 20 viewed from another direction;
FIG. 22 is a view in which the thawing part is separated from FIG. 20;
FIG. 23 is a view in which the thawing part is extracted and separated in FIG. 22;
FIG. 24 is a view in which the driving part is extracted from FIG. 22;
FIG. 25 is an enlarged view of a part of FIG. 21, in which portions of the first heating block and the second heating block are cut in a standby position;
FIG. 26 is a view showing a part of the cell thawing machine according to the second embodiment of the present invention in a state where the heating block is disposed at a heating position;
FIG. 27 is a view of FIG. 26 as viewed from above;
FIG. 28 is a view showing the arrangement relationship of sensors and heating blocks corresponding to the state of FIG. 26, showing a state in which the first heating block, the first heat insulating block and the first guide block are removed;
FIG. 29 is an operating state diagram of the cell thawing machine according to the second embodiment of the present invention, and is a partial cross-sectional view of the standby position when initially driven, as viewed from the C-C direction in FIG. 26;
FIG. 30 is an operating state diagram showing a state in which the heating block is preheated in the cell thawing machine according to the second embodiment of the present invention, and is a partial cross-sectional view as viewed from the C-C direction of FIG. 26;
FIG. 31 is an operating state diagram showing the process of introducing a container into the cell thawing machine according to the second embodiment of the present invention, and is a partial cross-sectional view as viewed from the C-C direction of FIG. 26;
FIG. 32 is an operating state diagram of the cell thawing machine according to the second embodiment of the present invention, and is a partial cross-sectional view of the state in which the container is heated in the heating position, as viewed from the direction C-C in FIG. 26;
FIG. 33 is an operating state diagram of the cell thawing machine according to the second embodiment of the present invention, and is a partial cross-sectional view of the process of removing a container from a standby position, as viewed from the C-C direction in FIG. 26; and
FIG. 34 is a block diagram showing the method for operating a cell thawing machine according to one embodiment of the present invention.

### [Modes of the Invention]

Hereinafter, with reference to the accompanying drawings, the exemplary embodiments of the present invention will be described in detail so that those skilled in the art can easily practice the present invention. The present invention may be embodied in many different forms and is not limited to the exemplary embodiments set forth herein. In order to clearly describe the present invention in the drawings, parts that are irrelevant to the description are omitted, and the same reference numerals are assigned to the same or similar components throughout the specification.

The cell thawing machines 100, 200 according to one embodiment of the present invention may heat and thaw a biological material contained in a container 10 in a low-temperature state (e.g., frozen state).

Herein, the biological material may include cells, which are the structural basic unit of an organism, and the container 10 may be a known laboratory container or container for medical use such as a vial, a beaker or a test tube.

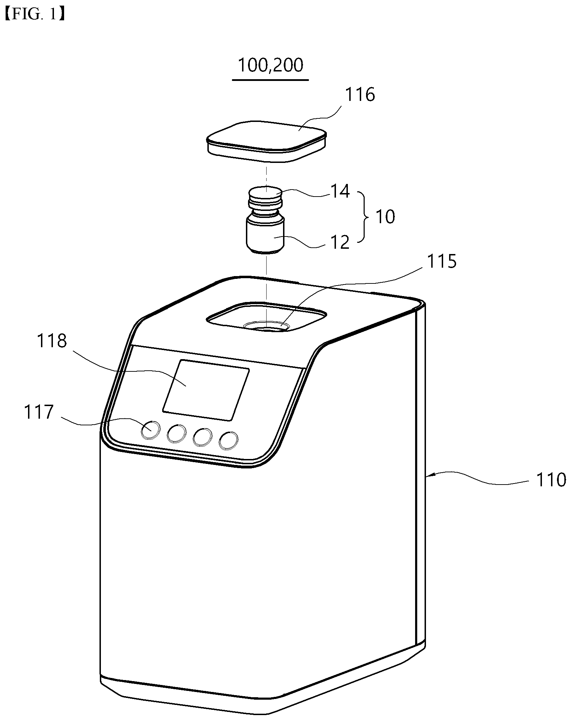

In addition, as illustrated in FIG. 1, the container 10 may include a container main body 12 in which the biological material is accommodated and a cap part 14 for covering the open top of the container main body 12, and approximately 6 mL or 10 mL of biological material may be contained in the container main body 12. However, the shape of the container 10 and the capacity of the biological material contained in the container main body 12 are not limited thereto and may be changed into various shapes depending on design conditions.

In this case, the cell thawing machines 100, 200 according to one embodiment of the present invention may heat the container 10 uniformly as a whole by heating the container 10 in a non-contact heating manner through radiant heat and/or convection heat, and it is possible to prevent cracks or thermal deformation of the container 10 that may occur during contact heating by conductive heat in advance.

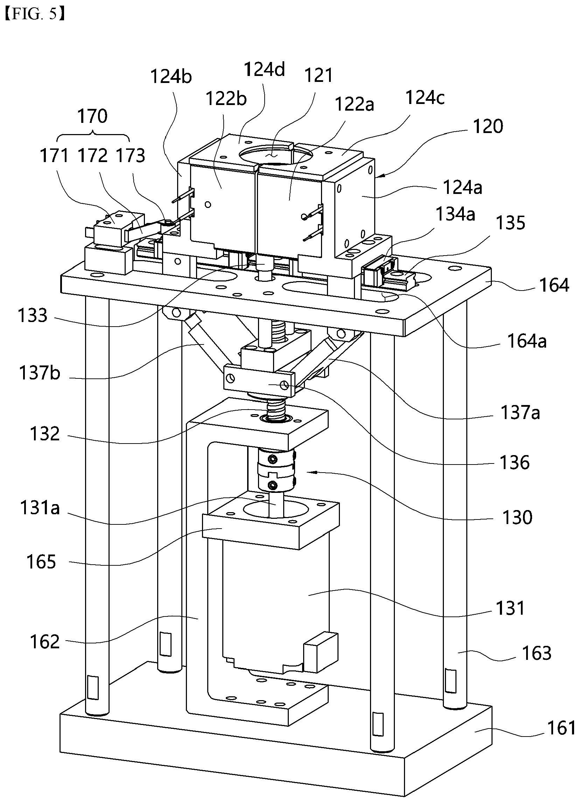

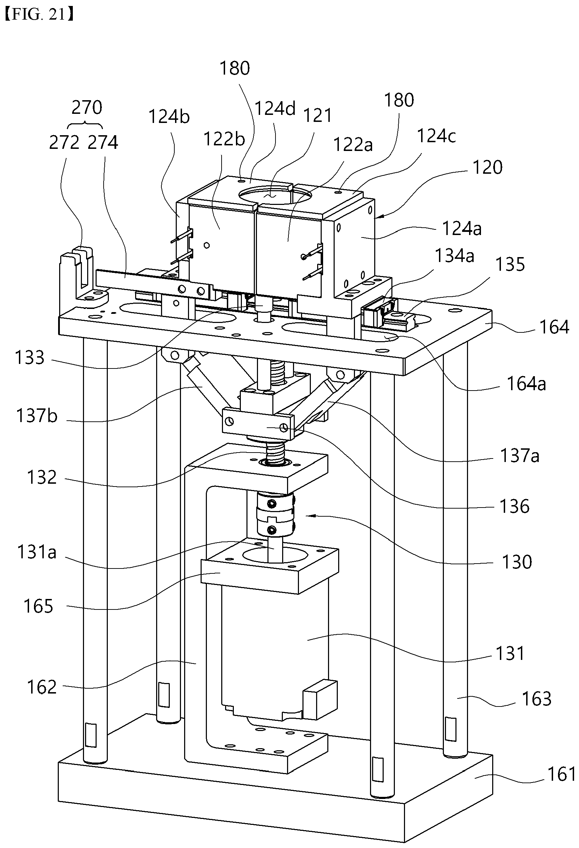

To this end, as illustrated in FIGS. 1 to 6 and 18 to 22, the cell thawing machines 100, 200 according to one embodiment of the present invention includes a housing 110, a thawing part 120, a driving part 130 and a control part 150.

As illustrated in FIGS. 1 to 3, the housing 110 may form an outer surface of the cell thawing machines 100, 200, and it may be formed in a box shape such that the thawing part 120, the driving part 130 and the control part 150 may be disposed therein.

Such a housing 110 may be composed of one member or may be composed of a plurality of members, and the plurality of members may be detachably coupled.

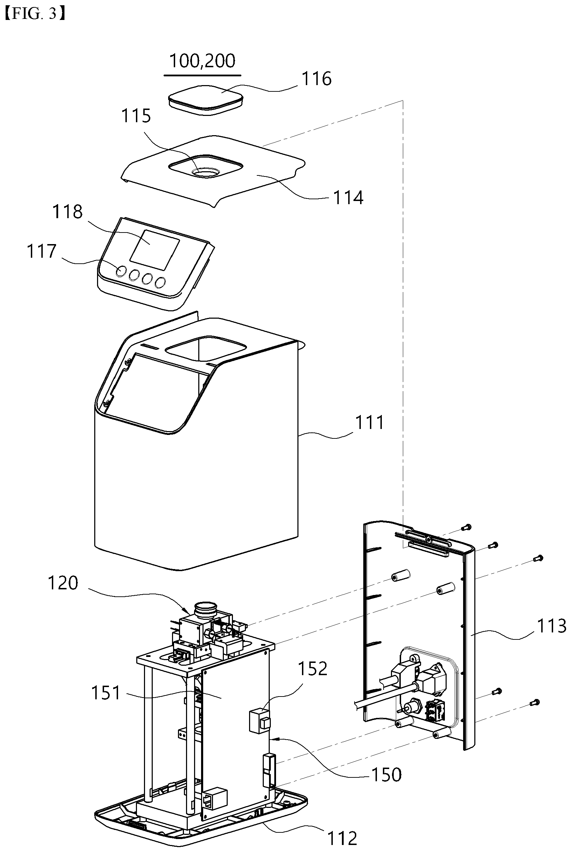

For example, as illustrated in FIG. 3, the housing 110 may include a box-shaped main body 111, a lower cover 112 for covering an open lower portion of the main body 111, a rear cover 113 for covering an open rear surface of the main body 111, and an upper cover 114 for covering an upper portion of the main body 111.

In this case, the thawing part 120, the driving part 130, and the control part 150 may be disposed inside the main body 111.

In this case, the housing 110 may include an inlet 115 which is formed to penetrate a predetermined area such that the container 10 can be inserted into the main body 111, and the inlet 115 may be covered through a cover 116.

Herein, the inlet 115 may be formed through the housing 110 so as to be located at a position corresponding to a heating space 121 to be described below.

For example, the inlet 115 may be formed to pass through the upper cover 114 to communicate with a heating space 121 formed in the thawing part 120 at a heating position to be described below. Through this, when the container 10 is inserted into the inlet 115, the cap part 14 of the container 10 may be exposed to the outside through the inlet 115, the remaining portion except for the cap part 14 may be inserted into the heating space 121.

In addition, as illustrated in FIG. 1, a manipulation part 117 for manipulating the operation of the cell thawing machines 100, 200 according to one embodiment of the present invention, and a display 118 for displaying the operating state of the cell thawing machines 100, 200 may be provided on one side of the housing 110.

Such a manipulation part 117 may be an interface for transmitting an input signal to the control part 150 through user manipulation.

Herein, the manipulation part 117 may be configured with a known press-type physical button or capacitive touch button.

In addition, the manipulation part 117 may be provided separately from the display 118, or may be provided in an integrated form with the display 118.

Moreover, the display 118 may output information on the overall operating state of the cell thawing machines 100, 200 operated through the control part 150 (e.g., the operating status such as the temperature of the heating blocks 122a, 122b, the surface temperature of the container 10, thawing or completion of thawing, or thawing progress time), date and time at the time of thawing, an error message such as sensor or heater failure, which will be described below, and the like.

Through this, the user may transmit input signals, such as operation and stop of the cell thawing machines 100, 200, thawing temperature adjustment and thawing time adjustment, to the control part 150 through manipulation of the manipulation part 117, and the display 118 may output information on the overall state of the cell thawing machines 100, 200 through the control part 150.

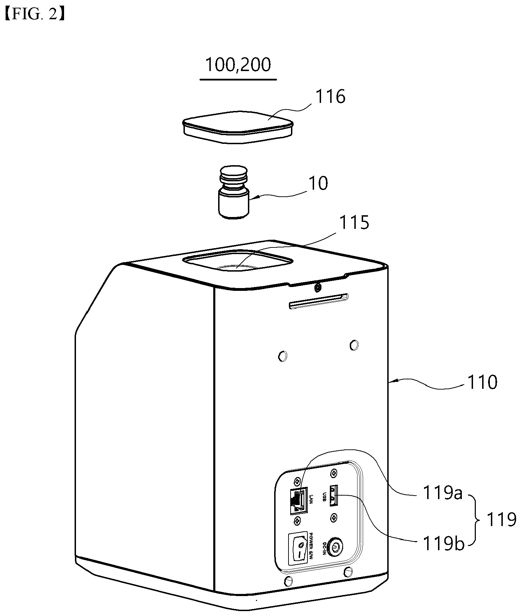

Moreover, as illustrated in FIG. 2, the cell thawing machines 100, 200 may include at least one communication port 119 that is provided to be exposed to the outside from one side of the housing 110, and the communication port 119 may be electrically connected to the control part 150.

For example, the communication port 119 may include a LAN port 119a for communication with communication device such as an external network device or a PC, and a USB port 119b for inputting or outputting data, and the communication port 119 may be mounted on the rear cover 113 so as to be electrically connected to the control part 150.

In this case, when the cell thawing machines 100, 200 according to one embodiment of the present invention are connected to the external network device or communication device such as a PC through the communication port 119, the control part 150 may be time-synchronized with communication device such as the external network device or PC.

For example, when the cell thawing machines 100, 200 are connected to a PC such as a laptop computer, the cell thawing machines 100, 200 may be synchronized with the date and time of the PC by using a synchronization program installed in the PC.

Through this, the cell thawing machines 100, 200 according to one embodiment of the present invention may be synchronized with the local time of the region where thawing is performed when in use, and the local time of the region may be output through the display 118.

As a result, the cell thawing machines 100, 200 according to one embodiment of the present invention may store history information about the exact time of thawing of the biological material contained in the container 10, and the user may confirm the exact thawing time of the biological material contained in the container 10 through the history information.

Additionally, information related to thawing may be stored in the cell thawing machines 100, 200 themselves or may be stored in a synchronization program of a communication device that is connected through the communication port 119.

In this case, although the information related to thawing stored in the cell thawing machines 100, 200 or the synchronization program may be stored by date, a thawing history may be stored for each thawing case, and the thawing history may include the local time of the region where the thawing is performed.

The thawing part 120 may heat the container 10 while the container 10 introduced into the housing 110 through the inlet 115 is accommodated in a heating space 121.

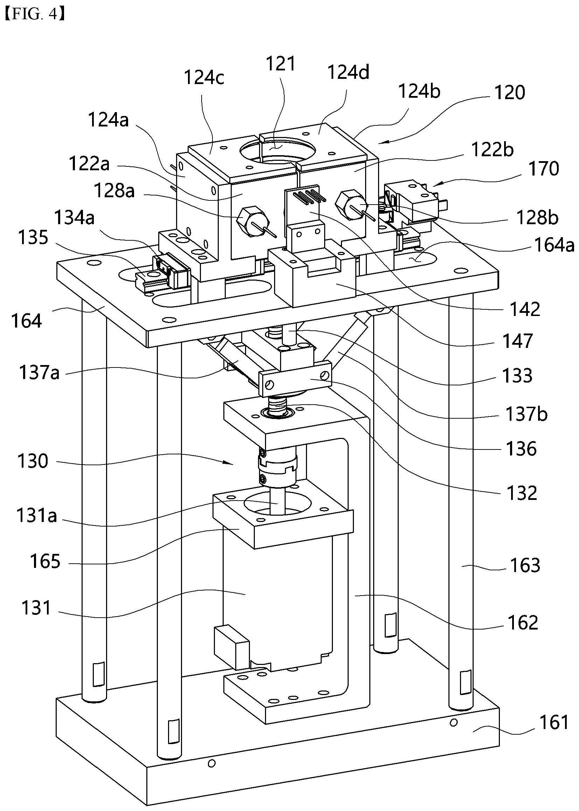

To this end, as illustrated in FIGS. 4 to 7 and 20 to 23, the thawing part 120 may include heating blocks 122a, 122b for forming a heating space 121 for accommodating and heating the container 10, heaters 123a, 123b which are installed in the heating blocks 122a, 122b so as to provide heat to the heating blocks 122a, 122b, and heat insulating blocks 124a, 124b which are coupled to the heating blocks 122a, 122b so as to surround the heaters 123a, 123b.

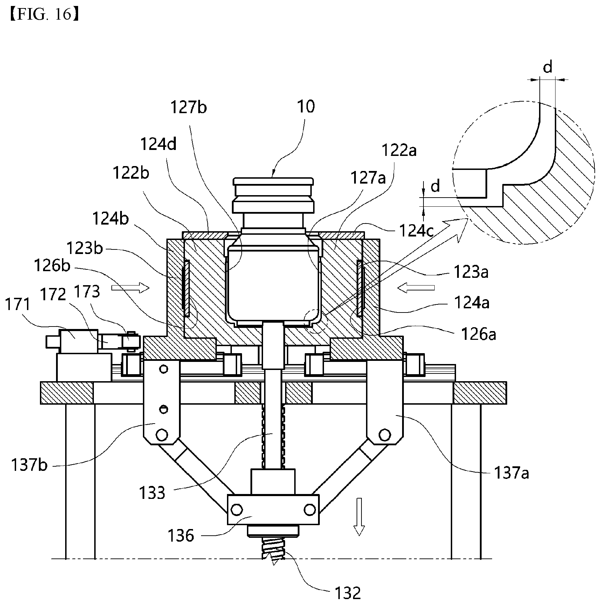

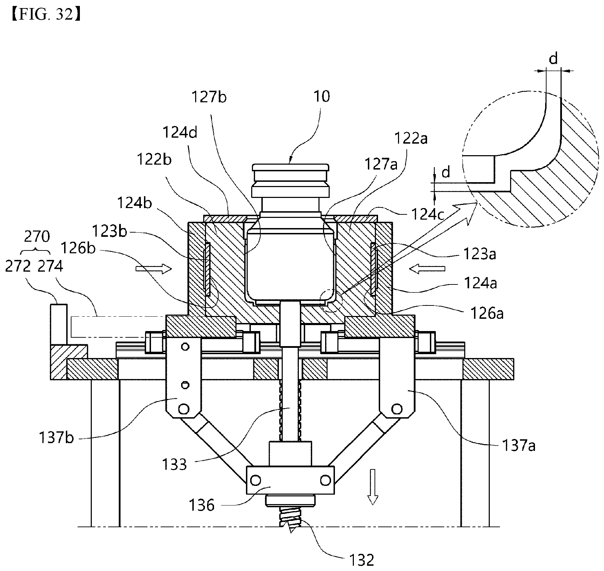

Accordingly, when the container 10 is introduced into the housing 110 through the inlet 115, the container 10 may be surrounded through the heating blocks 122a, 122b while being accommodated in the heating space 121 (refer to FIGS. 16 and 32).

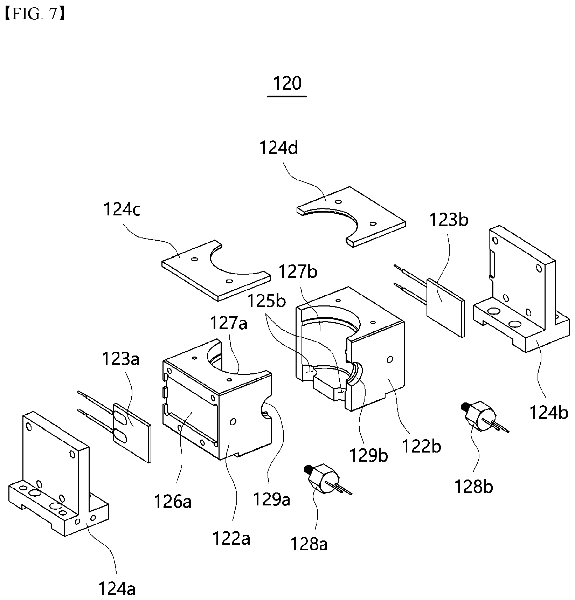

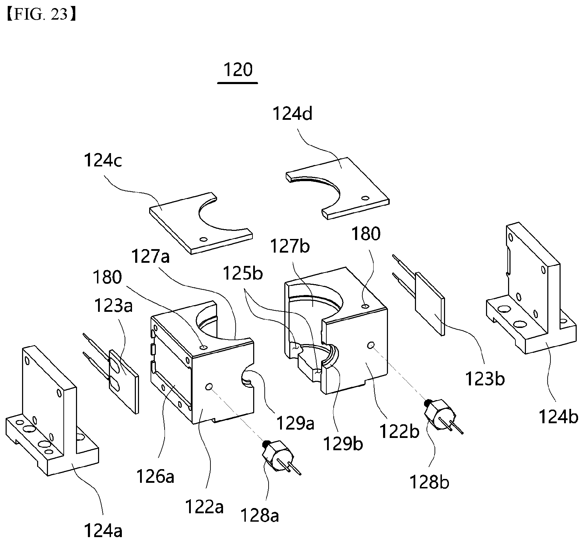

In this case, as illustrated in FIGS. 7 and 23, the heating blocks 122a, 122b may include arrangement grooves 126a, 126b which are formed to be drawn inwardly on one surface, and the heaters 123a, 123b may be inserted into the arrangement grooves 126a, 126b. In addition, while the heaters 123a, 123b are inserted into the arrangement grooves 126a, 126b, the heat insulating blocks 124a, 124b may be coupled to the heating blocks 122a, 122b so as to completely cover the arrangement grooves 126a, 126b.

In addition, the cell thawing machines 100, 200 according to one embodiment of the present invention may include auxiliary heat insulating blocks 124c, 124d to block heat transferred from the heaters 123a, 124b from being discharged to the outside, and the auxiliary heat insulating blocks 124c, 124d may be coupled to the heating blocks 122a, 122b to cover the upper surfaces of the heating blocks 122a, 122b.

Herein, the heating blocks 122a, 122b may be made of a material having thermal conductivity such as metal, and the heat insulating blocks 124a, 124b and the auxiliary heat insulating blocks 124c, 124d may be made of a material having heat insulating properties.

Accordingly, when the heaters 123a, 123b generate heat, the heating blocks 122a, 122b may be heated by the heat generated from the heaters 123a, 123b, and the heat generated from the heaters 123a, 123b may be concentrated toward the heating blocks 122a, 122b by limiting the direction of heat movement through the heat insulating blocks 124a, 124b and the auxiliary heat insulating blocks 124c, 124d, and the heat transferred from the heaters 123a, 123b to the heating blocks 122a, 122b may be transferred to the heating space 121.

As a result, the container 10 may be heated by the heat provided from the heating blocks 122a, 122b, and the biological material stored in the container 10 may be thawed by the heat.

Moreover, since the heat generated from the heaters 123a, 123b may be concentrated toward the heating blocks 122a, 122b by limiting the direction of heat movement through the heat insulating blocks 124a, 124b and the auxiliary heat insulating blocks 124c, 124d, it is possible to improve the energy consumption efficiency of the cell thawing machines 100, 200 according to one embodiment of the present invention.

In addition, since the container 10 accommodated in the heating space 121 may be heated by using the heat of the heating blocks 122a, 122b which is heated to a constant temperature by the heat transferred from the heaters 123a, 123b, the circumferential surface of the container 10 may be heated to a uniform temperature. Herein, the circumferential surface of the container 10 may be a circumferential surface including a side surface and a bottom surface of the container main body 12 excluding the cap part 14.

In this case, as illustrated in FIGS. 7 and 23, the thawing part 120 may include temperature sensors 128a, 128b which are installed on the heating blocks 122a, 122b to measure the temperatures of the heating blocks 122a, 122b. Herein, the temperature sensors 128a, 128b may be known contact-type temperature sensors such as thermocouples, resistance thermometers (RTDs) and thermistors, and the temperatures of the heating blocks 122a, 122b measured through the temperature sensors 128a, 128b may be transmitted to the control part 150.

Accordingly, the control part 150 controls the driving of the heaters 123a, 123b based on the temperature information measured by the temperature sensors 128a, 128b, it is possible to maintain the temperatures of the heating blocks 122a, 122b to a constant temperature. For example, the control part 150 may maintain the temperatures of the heating blocks 122a, 122b to be constant by controlling the driving of the heaters 123a, 123b through PID control.

Moreover, the control part 150 may prevent overheating of the heating blocks 122a, 122b by controlling the driving of the heaters 123a, 123b based on the temperature information measured by the temperature sensors 128a, 128b.

In the present invention, the heaters 123a, 123b may be ceramic heaters to secure reliability and improve the lifespan cycle of products even under operating conditions in which heating and cooling are repeatedly performed, but the present invention is not limited thereto, various known heaters may all be applied.

Meanwhile, the heating space 121 may be provided in a shape corresponding to the shape of the container 10, and the heating space 121 may be formed through at least two heating blocks 122a, 122b.

As a specific example, as illustrated in FIGS. 7 and 23, the heating blocks 122a, 122b may include a first heating block 122a and a second heating block 122b which are disposed such that surfaces thereof face each other, and the heating space 121 may be formed through a pair of opposing surfaces 127a, 127b which are formed to be drawn on surfaces facing each other in the first heating block 122a and the second heating block 122b.

Herein, the pair of opposing surfaces 127a, 127b may be formed to be drawn inward to have a shape corresponding to the circumferential surface of the container main body 12, and may be formed to have a shape corresponding to a side surface and lower surface of the container main body 12.

For example, when the container 10 is formed in a cylindrical shape, the pair of opposing surfaces 127a, 127b may include an arc-shaped curved surface and an arc-shaped horizontal surface connected to a lower edge of the curved surface, and when the container 10 is formed in the shape of a square pillar, the pair of opposing surfaces 127a, 127b may include a horizontal surface of a rectangular cross-section for connecting a bent surface having a substantially 'c' cross-sectional shape and a lower edge of the curved surface.

Accordingly, when the container 10 is introduced into the heating space 121 through the inlet 115, the circumferential surface including a side surface and a bottom surface of the container 10 may be completely surrounded by a pair of opposing surfaces 127a, 127b defining the heating space 121.

As a result, the container 10 accommodated in the heating space 121 may be uniformly heated on the circumferential surface including a lower surface along with a side surface by the heat provided from the first heating block 122a and the second heating block 122b.

In this case, each of the first heating block 122a and the second heating block 122b may include arrangement grooves 126a, 126b which are formed to be drawn inward on one surface thereof, respectively, and the heaters 123a, 123b may include a first heater 123a and a second heater 123b that are disposed to be inserted into the arrangement groove 126a formed in the first heating block 122a and the arrangement groove 126b formed in the second heating block 122b, respectively.

In addition, the heat insulating blocks 124a, 124b may include a first heat insulating block 124a and a second heat insulating block 124b, and the first heat insulating block 124a may be coupled to the first heating block 122a so as to completely cover the arrangement groove 126a while the first heater 123a is inserted into the arrangement groove 126a of the first heating block 122a, and the second heat insulating block 124b may be coupled to the second heating block 122b so as to completely cover the arrangement groove 126b while the second heater 123b is inserted into the arrangement groove 126b of the second heating block 122b.

Moreover, the auxiliary heat insulating blocks 124c, 124d may include a first auxiliary heat insulating block 124c which is fastened to an upper surface of the first heating block 122a, and a second auxiliary heat insulating block 124d which is fastened to an upper surface of the second heating block 122b.

In addition, the temperature sensors 128a, 128b may include a first temperature sensor 128a which is installed in the first heating block 122a to measure the temperature of the first heating block 122a, and a second temperature sensor 128b which is installed in the second heating block 122b to measure the temperature of the second heating block 122b.

Accordingly, the heat generated from the first heater 123a may heat the first heating block 122a, and the heat generated from the second heater 123b may heat the second heating block 122b, and the control part 150 may control the driving of the first heater 123a and the second heater 123b based on the temperature information measured by the first temperature sensor 128a and the second temperature sensor 128b such that it is possible to maintain the temperatures of the first heating block 122a and the second heating block 122b to be constant.

In this case, the control part 150 may control the operations of the first heater 123a and the second heater 123b together based on the temperature information measured through the first temperature sensor 128a and the second temperature sensor 128b, or the control part 150 may individually control the operations of the first heater 123a and the second heater 123b based on the temperature information measured through the first temperature sensor 128a and the second temperature sensor 128b.

Through this, even if the heating blocks 122a, 122b include the first heating block 122a and the second heating block 122b in the cell thawing machines 100, 200 according to one embodiment of the present invention, the temperatures of the first heating block 122a and the second heating block 122b may be precisely controlled through the control of the control part 150.

Accordingly, since the first heating block 122a and the second heating block 122b may be maintained at the same temperature without temperature deviation through the control part 150, the circumferential surface of the container 10 including a side surface and lower surface in the heating space 121 may be uniformly heated.

Moreover, when the control part 150 individually controls the driving of the first heater 123a and the second heater 123b, the user may immediately check an abnormality of the corresponding part based on the information output through the display 118, and take appropriate action on the corresponding part in which the abnormality has occurred.

For example, when any one of the heaters 123a, 123b that are respectively installed in the first heating block 122a and the second heating block 122b or any one of the temperature sensors 128a, 128b that are respectively installed the first heating block 122a and the second heating block 122b does not operate normally, the user may immediately check an abnormality of the corresponding part based on the information output through the display 118, and take appropriate action on the corresponding part in which the abnormality has occurred.

Meanwhile, in the cell thawing machines 100, 200 according to one embodiment of the present invention, while the container 10 accommodated in the heating space 121 does not contact the heating block 122a, 122b, the container 10 may be heated by the heat transferred from the heating block 122a., 122b in a non-contact manner.

For example, while the container 10 is inserted into the heating space 121, the circumferential surface of the container 10 may be spaced apart by a certain interval from the opposing surface 127a of the first heating block 122a and the opposing surface 127b of the second heating block 122b forming the heating space 121.

In this case, the lower portion of the container 10 may be supported through support pins 133 whose ends protrude from a portion forming the bottom surface of the heating space 121 toward the heating space 121 at a predetermined height.

That is, as illustrated in FIGS. 16 and 32, while the container 10 is inserted into the heating space 121 and the lower portion of the container 10 is supported through the support pins 133, a gap (d) may be formed between the pair of opposing surfaces 127a, 127b and the outer surface of the container 10.

Accordingly, the heat stored in the first heating block 122a and the second heating block 122b may be transferred to the relatively low-temperature container 10 through convection and/or radiation instead of conduction.

As a result, since the cell thawing machines 100, 200 according to one embodiment of the present invention may heat the circumferential surface of the container 10 through heat transfer using convection and/or radiation, the circumferential surface of the container 10 may be uniformly heated through convective heat and/or radiant heat transmitted from the heating blocks 122a, 122b.

That is, compared to the case where the container 10 and the heating blocks 122a, 122b are in direct contact and the container 10 is heated through conduction heat, the cell thawing machines 100, 200 according to one embodiment of the present invention may uniformly heat the circumferential surface of the container through convective heat and/or radiant heat, and thus, it is possible to prevent the concentration of heat in a local location of the container 10.

Moreover, in the cell thawing machines 100, 200 according to one embodiment of the present invention, even if the container 10 is made of a glass or plastic material that is vulnerable to high-temperature heat, the first heating block 122a and the second heating block 122b do not come into direct contact with the container 10, and thus, it is possible to prevent cracks or thermal deformation of the container that may occur due to heat when in direct contact with a high-temperature obj ect.

In the present invention, the gap (d) between the pair of opposing surfaces 127a, 127b forming the heating space 121 and the outer surface of the container 10 may be 0.2 mm to 0.3 mm, but the present invention is limited thereto, and the size of the gap (d) may be appropriately changed depending on the overall size of the container 10.

In this case, at least any one of the first heating block 122a and the second heating block 122b may be linearly moved reciprocally in a straight line, and the heating space 121 may be formed when the first heating block 122a and the second heating block 122b are disposed to be located in nearby positions through the linear movement of at least any one of the first heating block 122a and the second heating block 122b.

That is, the driving part 130 may linearly move the heating blocks 122a, 122b in left and right directions. For example, as described above, when the heating blocks 122a, 122b include a first heating block 122a and a second heating block 122b, the driving part 130 may linearly move at least any one of the first heating block 122a reciprocally in the left and right directions.

As a non-limiting example, when the heating blocks 122a, 122b include a first heating block 122a and a second heating block 122b, the driving part 130 may linearly move the first heating block 122a and the second heating block 122b reciprocally in the left and right directions, respectively.

In addition, when the heating space 121 is formed between the first heating block 122a and the second heating block 122b through linear movement of at least one of the first heating block 122a and the second heating block 122b, the driving part 130 may protrude an end of the support pin 133 at a predetermined height from a portion forming the bottom surface of the heating space 121.

Accordingly, the end of the support pin 133 protruding at a predetermined height from the portion forming the bottom surface of the heating space 121 may support a lower portion of the container 10.

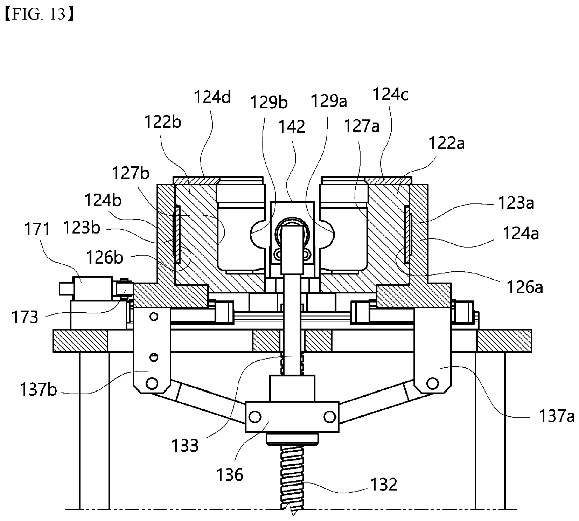

Through this, as illustrated in FIGS. 16, 30 and 32, the cell thawing machines 100, 200 according to one embodiment of the present invention may be changed to a heating position in which the first heating block 122a and the first heating block 122a are disposed to be close to each other through the driving of the driving part 130 and an end of the support pin 133 protrudes toward the heating space 121, and a standby position in which the first heating block 122a and the second heating block 122b are disposed to be spaced apart from each other at a gap through the driving of the driving part 130 and the end of the support pin 133 is raised to a relatively higher position than the heating position as illustrated in FIGS. 13, 15, 17, 29, 31 and 33.

Accordingly, as illustrated in FIGS. 16 and 32, when the first heating block 122a and the second heating block 122b are located in the heating position, a heating space 121 may be formed between the opposing surface 127a of the first heating block 122a and the opposing surface 127b of the second heating block 122b to accommodate the container 10 and heat the container 10, and the support pin 133 may descend to protrude a certain length from the portion forming the bottom surface of the heating space 121 inside the heating space 121.

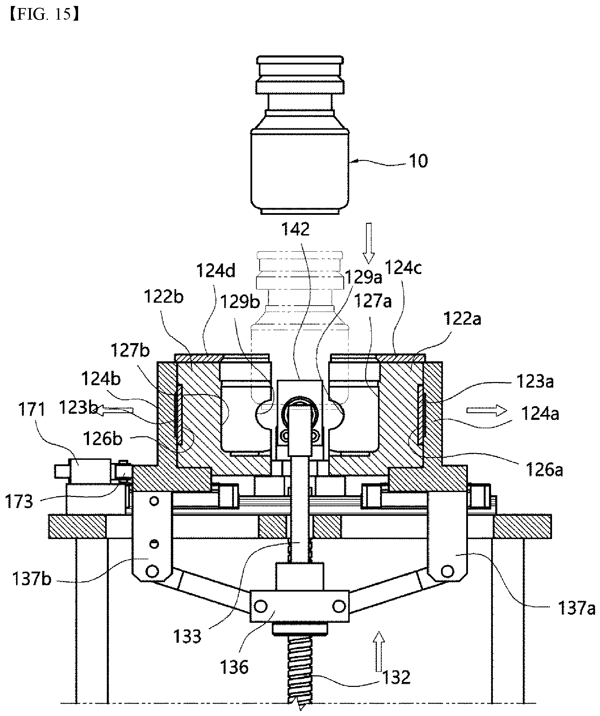

In addition, as illustrated in FIGS. 15, 17, 31 and 33, when the first heating block 122a and the second heating block 122b are located in the standby position, the gap between the opposing surface 127a of the first heating block 122a and the opposing surface 127b of the second heating block 122b widens such that the support pin 133 may rise to a certain height. Through this, the user may place the container 10 at an end of the support pin 133 protruding at a certain height between the first heating block 122a and the second heating block 122b or remove the container 10 whose lower portion is supported by the support pins 133 between the first heating block 122a and the second heating block 122b.

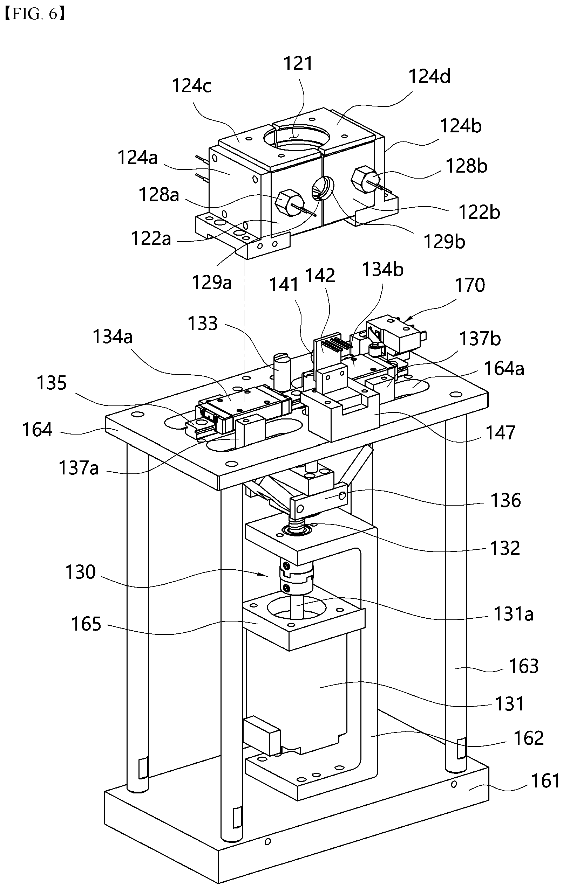

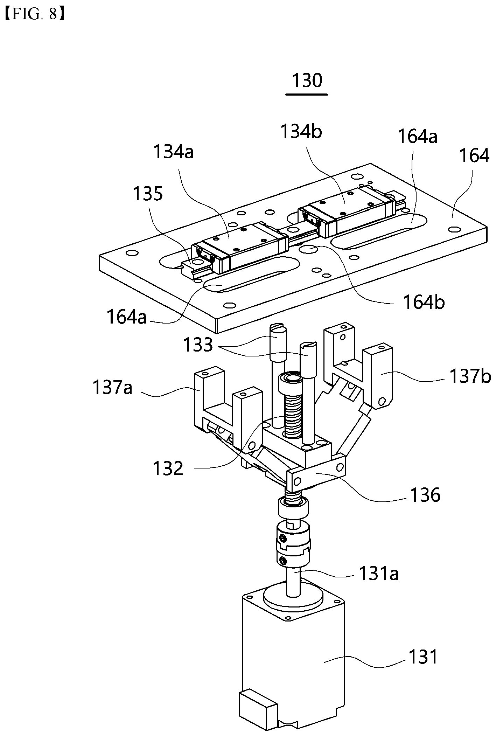

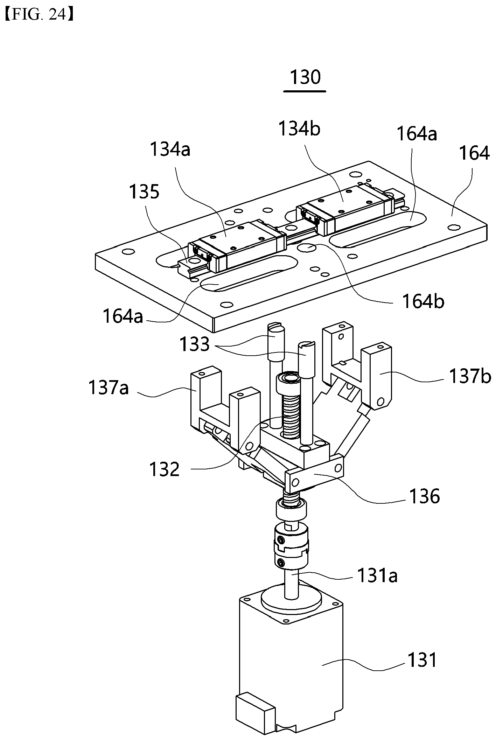

To this end, as illustrated in FIGS. 6, 8, 22 and 24, the driving part 130 may include a motor for providing a driving force, a rotating member 132 which is axially coupled to a rotation shaft 131a of the motor 131, and a moving member 136 which ascends and descends along the rotating member 132 when the rotating member 132 rotates.

In addition, the driving part 130 may include support pins 133 which are coupled to the moving member 136 so as to support a lower portion of the container 10 by moving up and down together with the moving member 136, and a power transmission member for interconnecting the rotating member 132 and the thawing part 120 so as to linearly move the first heating block 122a and the second heating block 122b in left and right directions, respectively, through a driving force of the motor 131.

Moreover, the power transmission member may include guide blocks 134a, 134b which are fixedly coupled to the heat insulating blocks 124a, 124b, guide rails 135 for guiding linear movement of the guide blocks 134a, 134b, and link members 137a, 137b for linking the guide blocks 134a, 134b and the moving member 136 to each other.

In this case, the heating blocks 122a, 122b may include through-holes 125a, 125b through which the support pins 133 pass such that the support pins 133 can smoothly ascend and descend when the moving member 136 moves up and down.

As a specific example, the guide blocks 134a, 134b may include a first guide block 134a which is fixedly coupled to the first heat insulating block 124a and a second guide block which is fixedly coupled to the second heat insulating block 124b, and the first guide block 134a and the second guide block 134b may be slidably coupled to the guide rail 135.

In addition, the link members 137a, 137b may include a first link member 137a for interconnecting the moving member 136 and the first guide block 134a, and a second link member 137b for linking the moving member 136 and the second guide block 134b to each other.

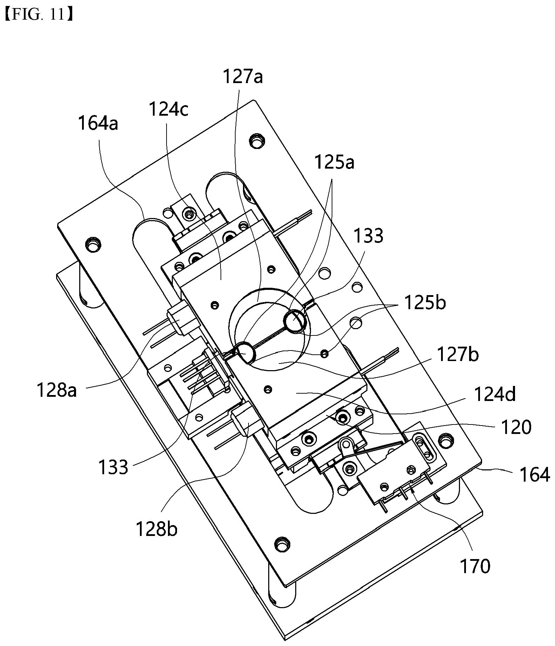

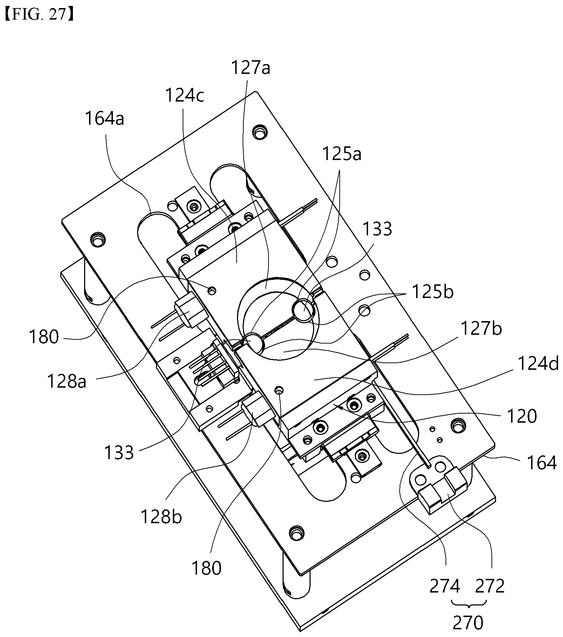

Moreover, as illustrated in FIGS. 11 and 27, the through- holes 125a, 125b may include a first through-hole 125a which is formed to penetrate the first heating block 122a, and a second through-hole 125b which is formed to penetrate the second heating block 122b, and the first through-hole 125a and the second through-hole 125b may be joined together to form one through-hole.

In this case, the cell thawing machines 100, 200 according to one embodiment of the present invention may further include a base 161, a mounting table 162, a support bar 163 and a support 164 that are disposed inside the housing 110.

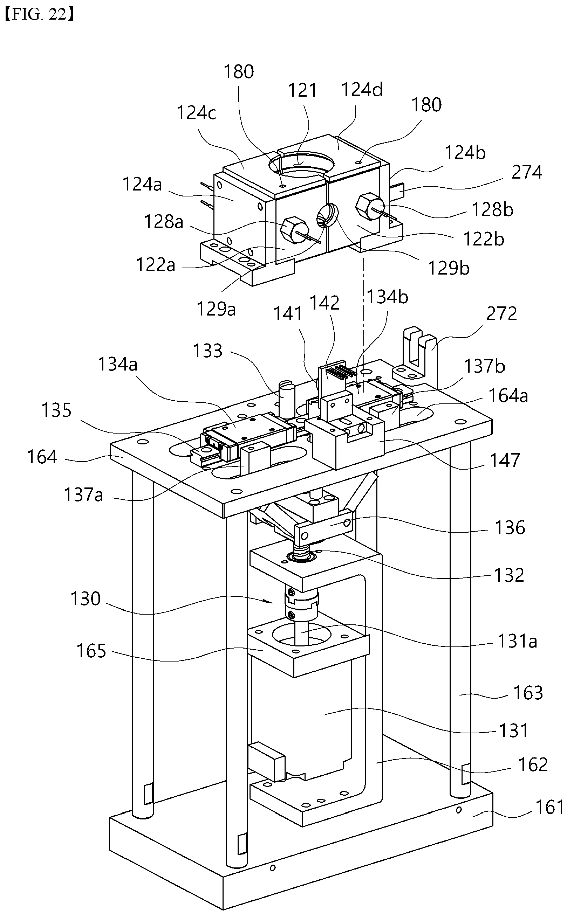

That is, as illustrated in FIGS. 6 and 22, a mounting table 162 may be fixedly coupled to an upper surface of the base 161, and the motor 131 may be fixedly coupled to one side of the mounting table 162 via a coupling table 165, and the rotating member 132 which is axially coupled to a rotating shaft 131a of the motor 131 may be rotatably coupled to the mounting table 162.

In addition, the plate-shaped support 164 having a predetermined area may be disposed at a certain interval from an upper portion from the base 161 through at least one support bar 163 having a predetermined length and one end of which is fixedly coupled to the base 161, and the guide rail 135 may be fixedly coupled to one surface of the support 164.

Moreover, the first guide block 134a and the second guide block 134b may be slidably coupled to the guide rail 135, and the first heat insulating block 124a may be fixedly coupled to the first guide block 134a, and the second heat insulating block 124b may be fixedly coupled to the second guide block 134b.

Herein, the rotating member 132 may be a screw bar having a predetermined length and having one end axially coupled to a rotating shaft 131a of the motor 131, and the moving member 136 may be screw-coupled to the rotating member 132 to be movable, and one end of the support pin 133 may be fixed to the moving member 136 such that the support pin 133 can be ascended and descended together when the moving member 136 moves up and down.

In addition, the support 164 may include a long first through-hole 164a which is formed to penetrate such that the first link member 137a and the second link member 127b can pass through, respectively, and a second through-hole 164b which is formed to penetrate such that the support pins 133 can pass through.

Moreover, both ends of the first link member 137a may be linked to the rotating member 132 and the first guide block 134a, respectively, and both ends of the second link member 137b may be linked to the rotating member 132 and the second guide block 134b, respectively.

Accordingly, when the rotating member 132 which is axially coupled to the rotating shaft 131a rotates when the motor 131 is driven, as illustrated in FIGS. 13 to 17 and 29 to 33, the moving member 136 may be moved up and down by screw movement along the rotating member 132, and the support pins 133 may be moved up and down together with the moving member 136.

In addition, the first guide block 134a and the second guide block 134b which are each linked to the moving member 136 may be linearly moved in left and right directions along the guide rail 135 through ascending and descending of the moving member 136.

As a result, the first heat insulating block 124a and the second heat insulating block 124b, which are fixedly coupled to the first guide block 134a and the second guide block 134b, respectively, may be linearly moved in left and right directions in the same manner as the first guide block 134a and the second guide block 134b, and the first heating block 122a and the second heating block 122b, which are fixedly coupled to the first heat insulating block 124a and the second heat insulating block 124b, respectively, may also be linearly moved in the left and right directions in the same way.

Through this, in the cell thawing machines 100, 200 according to one embodiment of the present invention, the first heating block 122a, the second heating block 122b and the support pins 133 may be changed to a heating position in which the first heating block 122a and the second heating block 122b are disposed to be close to each other through the driving of the driving part 130 and an end of the support pin 133 protrudes at a certain height from a portion forming a bottom surface of the heating space 121, and may be changed to a standby position in which the first heating block 122a and the second heating block 122b are disposed to be spaced apart from each other at an interval through the driving of the driving part 130 and an end of the support pin 133 rises to a relatively higher position than the heating position.

Moreover, when the cell thawing machines 100, 200 according to one embodiment of the present invention are changed from the standby position to the heating position through the control of the control part 140, a lower portion of the container 10, which is supported through the support pins 133 in the heating space 121, may be spaced apart by a certain interval from a portion forming a bottom surface of the heating space 121.

Accordingly, the container 10 disposed in the heating space 121 may be disposed in the heating space 121 in a state where the circumferential surface does not come into contact with the heating blocks 122a, 122b.

The drawings and description illustrate and describe that the rotational movement of the motor 131 is changed to the linear movement of the first guide block 134a and the second guide block 134b through mutual coupling of the rotating member 132, the moving member 136 and the link members 137a, 137b, but the present invention is not limited thereto, and various known structures such as a ball screw structure may all be applied as long as rotational movement can be converted into linear movement.

In this case, the cell thawing machines 100, 200 according to one embodiment of the present invention may further include a position detecting means for detecting the positions of the heating blocks 122a, 122b.

For example, in the cell thawing machine 100 according to one embodiment of the present invention, the position detecting means may be a limit switch 170 for detecting the positions of the heating blocks 122a, 122b through contact with the heat insulating blocks 124a, 124b.

As illustrated in FIG. 6, the limit switch 170 may be installed on one surface of the support 164, and may detect the position of the second heating block 122b through contact with the second heat insulating block 124b, and the information detected through the limit switch 170 may be provided to the control part 150.

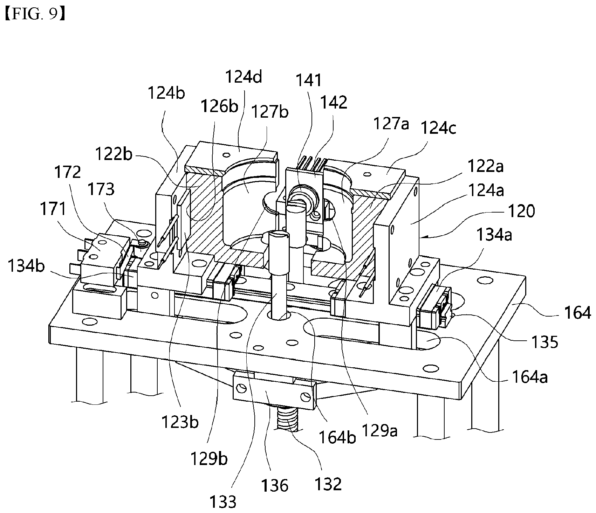

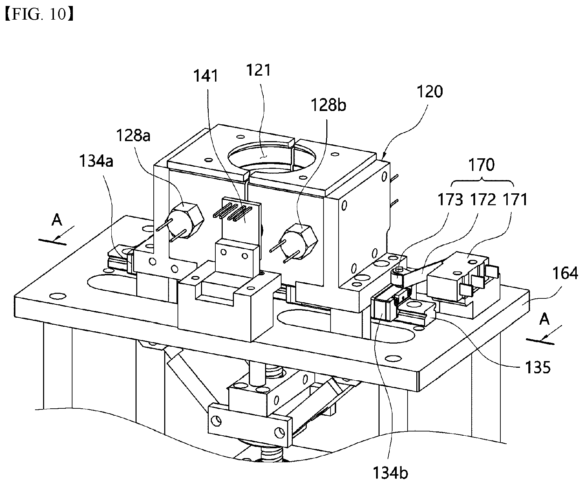

As a specific example, as illustrated in FIGS. 9 and 10, the limit switch 170 may include a switch box 171 which is fixedly installed on one surface of the support 164, an operating lever 172 which performs a hinge operation on one side of the switch box 171, and a roller 173 which is rotatably coupled to one end of the operating lever 172.

Accordingly, in the limit switch 170, when the roller 173 contacts the second heat insulating block 124b and the operating lever 172 operates, the switch box 171 may be operated, and information on whether the switch box 171 is operated may be transmitted to the control part 150.

Specifically, as described above, when the first heating block 122a and the second heating block 122b are changed from the heating position to the standby position through the driving of the driving part 130, the second heat insulating block 124b may move together with the second heating block 122b.

Through this, the second heat insulating block 124b may come into contact with the roller 173, and as illustrated in FIG. 9, when the second heating block 122b is completely moved to the standby position, the operating lever 172 may be operated such that the switch box 171 can be operated.

In this case, the control part 150 determines that the second heating block 122b is correctly positioned in the standby position and controls the driving of the motor 131 such that it is possible to prevent the second heating block 122b from moving excessively.

In addition, as illustrated in FIG. 10, when the first heating block 122a and the second heating block 122b are changed from the standby position to the heating position through the driving of the driving part 130, the second heat insulating block 124b is moved together with the second heating block 122b such that the contact state between the second heat insulating block 124b and the roller 173 can be released.

The drawings and description exemplify a hinge roller-type limit switch as a position detection means for detecting the position of the second heating block 122b, but the present invention is not limited thereto, and various known limit switches, such as lever-type limit switches and pin-type limit switches, may all be employed.

As another example, in the cell thawing machine 200 according to one embodiment of the present invention, the position detection means may further include a position detection sensor 270 for detecting the positions of the heating blocks 122a, 122b that linearly move in left and right directions through the driving of the driving part 130.

Such a position detection sensor 270 may be a non-contact position detection sensor.

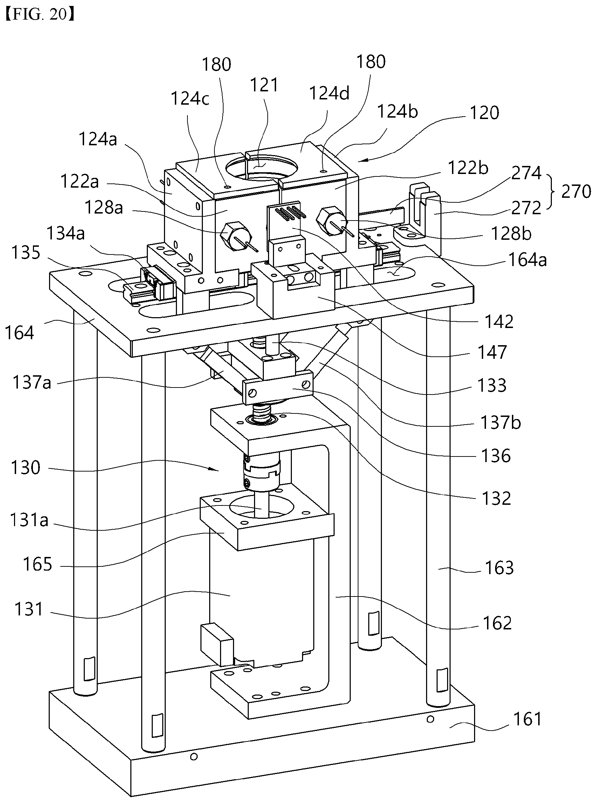

For example, the non-contact position detection sensor 270 may be a known photo-microsensor. As a non-limiting example, as illustrated in FIGS. 20 to 22, the non-contact position detection sensor 270 may include a photosensor 272 which is installed on one surface of the support 164, and a detection bar 274, one end of which is fixedly installed in the second heat insulating block 124b.

Accordingly, when the detection bar 274 approaches the photosensor 272 through linear movement of the second heating block 122b and the second heat insulating block 124b, the photosensor 272 may detect the position of the second heating block 122b through the detection bar 274, and the information detected through the position detection sensor 270 may be provided to the control part 150.

As a result, the control part 150 may accurately detect the position of the second heating block 122b through the non-contact position detection sensor 270, and may accurately check the origin of the motor 131.

Specifically, as described above, when the first heating block 122a and the second heating block 122b are changed from the heating position to the standby position through the driving of the driving part 130, the second heat insulating block 124b may move together with the second heating block 122b.

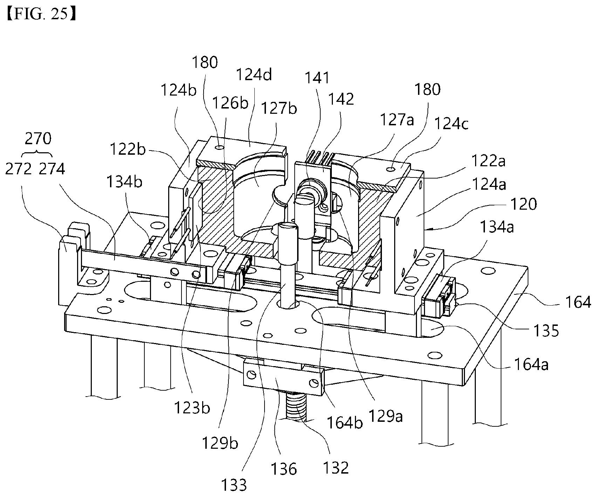

Through this, the detection bar 274, which is fixed to the second heat insulating block 124b, may move toward the photosensor 272, and as illustrated in FIG. 25, when the second heating block 122b is completely moved to the standby position, a partial length of the detection bar 274 may enter a groove portion that is formed in the photosensor 272.

In this case, the control part 150 determines that the second heating block 122b is correctly located in the standby position and controls the driving of the motor 131 such that it is possible to prevent the second heating block 122b from moving excessively.

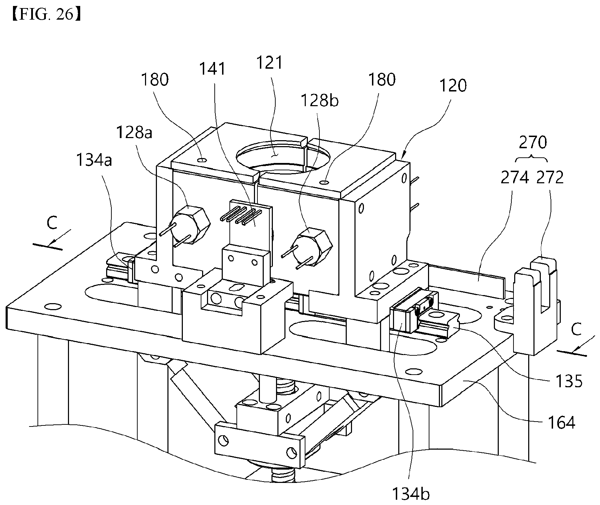

In addition, as illustrated in FIG. 26, when the first heating block 122a and the second heating block 122b are changed from the standby position to the heating position through the driving of the driving part 130, the detection bar 274, which is fixed to the second heat insulating block 124b, may move away from the photosensor 272, as the second heat insulating block 124b moves together with the second heating block 122b.

The drawings and description exemplify a non-contact sensor as a method for detecting the position of the second heating block 122b, but the present invention is not limited thereto, and various known position detection sensors may be employed as long as the position of the heating block 122 can be detected.

Meanwhile, the thawing part 120 may further include a sensor 141 for checking the state of the container 10 disposed in the heating space 121. For example, the sensor 141 may detect the surface temperature of the container 10 disposed in the heating space 121 and provide the detected result to the control part 150.

Herein, the sensor 141 may be an infrared temperature sensor so as to measure the surface temperature of the container 10 in a non-contact manner, and the sensor 141 may measure the surface temperature of the container 10 disposed in the heating space 121 while being fixed to the mounting member 142.

Accordingly, the control part 150 may output information about the container 10 detected through the sensor 141 to the display 118.

As a result, the user may check the thawing process of the biological material contained in the container 10 in real time through the information output from the display 118, and may monitor whether the biological material is thawing within an appropriate temperature range.

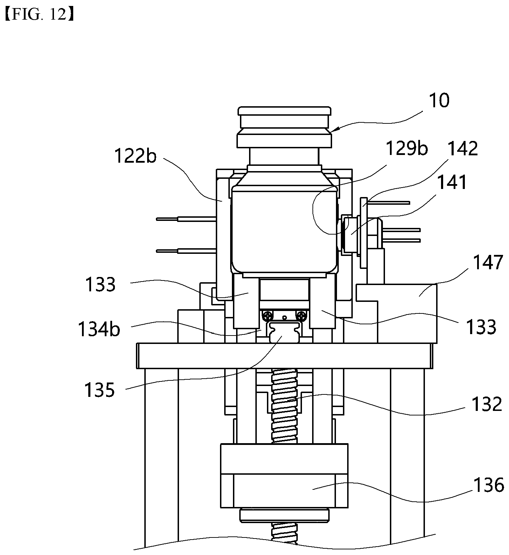

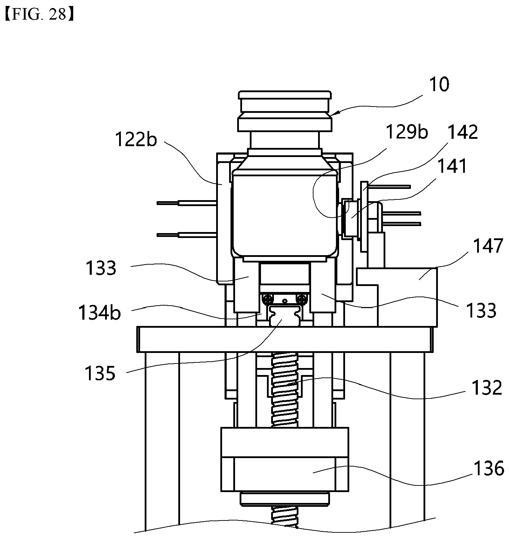

To this end, as illustrated in FIGS. 9, 12, 25 and 28, the sensor 141 may be coupled to the mounting member 142 so as to check the state of the container 10 disposed in the heating space 121, and the mounting member 142 may be coupled to a fixing table 147 that is fixedly coupled to the support 164.

In this case, the heating blocks 122a, 122b may include placement holes 129a, 129b which are formed in a shape corresponding to the sensor 141 such that the sensor 141 can enter the heating space 121 side.

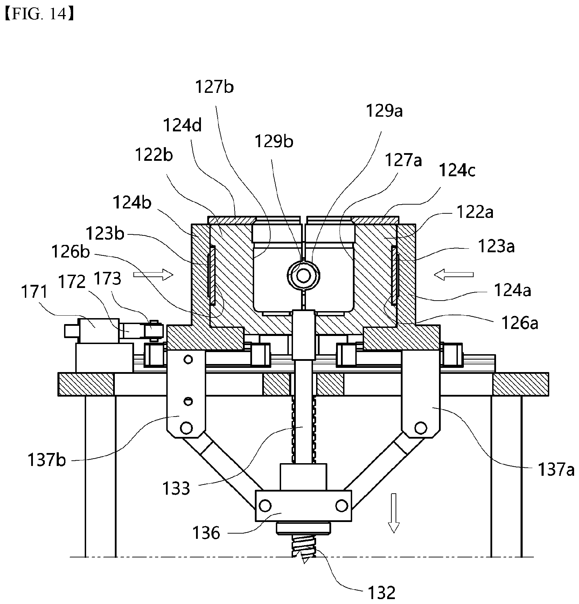

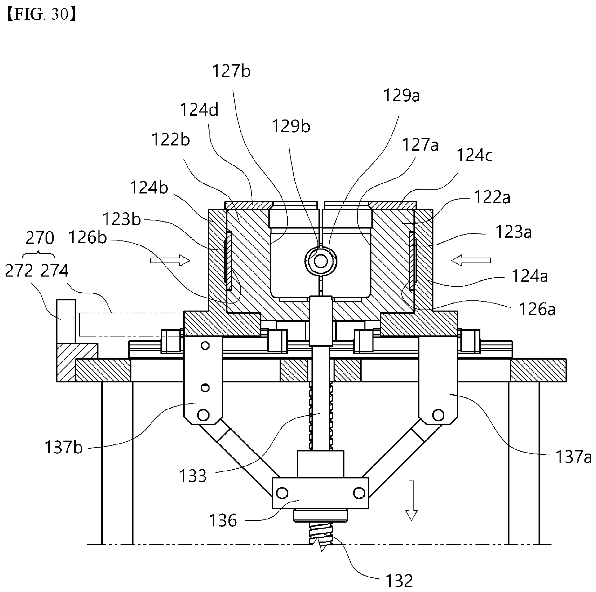

That is, as illustrated in FIGS. 14 and 30, the placement holes 129a, 129b may be formed to pass through the first heating block 122a and the second heating block 122b, respectively, and the placement holes 129a, 129b may be formed by joining a portion 129a that is formed in the first heating block 122a and a portion 129b that is formed in the second heating block 122b.

Accordingly, when the thawing part 120 is located at the heating position, the sensor 141 does not come into contact with the container 10 disposed in the heating space 121 through the placement holes 129a, 129b, and may measure the surface temperature of the container 10 at a location very close to the container 10.

Moreover, the temperature information measured by the sensor 141 may be used as information for detecting whether the container 10 is disposed in the heating space 121.

In the above description, an infrared temperature sensor is exemplified as the sensor 141, but the present invention is not limited thereto, and various known sensors may all be employed, and depending on the type of sensor employed, the information of the container 10 measured may vary.

Moreover, the mounting member 142 to which the sensor 141 is fixed may be detachably coupled to the fixing table 147. Accordingly, when the sensor 141 needs to be replaced, the sensor 141 may be easily replaced by separating the mounting member 142 from the fixing table 147.

The control part 150 may control the overall driving of the cell thawing machines 100, 200 according to one embodiment of the present invention.

As illustrated in FIG. 3, the control part 150 may include a circuit board 151 and a chipset 152 such as an MCU that is mounted on the circuit board 151, and the control part 150 may control all operations of the thawing part 120, the driving part 130, the sensing part 140, the display 118, the manipulation part 117 and the position detection means described above.

In this case, when heating the container 10 accommodated in the heating space 121 at the heating position, the control part 150 may control the driving of the driving part 130 so as to heat the container while the first heating block 122a and the second heating block 122b are in contact with each other, but it may also control the driving of the driving part 130 so as to heat the container 10 while the first heating block 122a and the second heating block 122b do not come into contact with each other.

That is, the control part 150 may set the heating position such that a predetermined gap is formed in the remaining portion except for portions where the opposing surfaces 127a, 127b for forming the heating space 121 are formed among the surfaces of the first heating block 122a and the second heating block 122b facing each other.

Through this, the cell thawing machines 100, 200 according to one embodiment of the present invention may prevent a load that may occur in the motor 131 in order to maintain contact between the first heating block 122a and the second heating block 122b at the heating position.

Additionally, in the process of thawing the biological material contained in the container 10 at the heating position, the cell thawing machines 100, 200 according to one embodiment of the present invention may discharge water vapor or moisture generated from the surface of the container 10 to the outside through the gap. Through this, the biological material contained in the container 10 may be thawed more smoothly.

Moreover, after preheating the heating blocks 122a, 122b to a predetermined temperature through driving of the heaters 123a, 123b, the control part 150 may start thawing the biological material contained in the container 10.

As described above, the control part 150 may maintain the temperatures of the heating blocks 122a, 122b to be constant through PID control, and when the heating blocks 122a, 122b are provided in plurality, the control part 150 may individually control each of the heating blocks 122a, 122b.

Herein, the preheating temperature of the heating blocks 122a, 122b may be the same as the thawing temperature for thawing the biological material contained in the container 10 or may be a relatively higher temperature than the thawing temperature.

In addition, the control part 150 may maintain the temperatures of the heating blocks 122a, 122b to be constant while the container 10 which is introduced into the heating space 121 is completely thawed and the heating blocks 122a, 122b are changed to the standby position.

Through this, the cell thawing machines 100, 200 according to one embodiment of the present invention may be changed between a heating position and a standby position while the heating blocks 122a, 122b are continuously maintained at a constant temperature.

Accordingly, when the cell thawing machines 100, 200 according to one embodiment of the present invention are used, the user may sequentially introduce and remove a plurality of containers 10 into the heating space 121 to perform thawing, thereby minimizing the waiting time increase work productivity.

Meanwhile, the cell thawing machine 200 according to one embodiment of the present invention may conveniently perform qualification evaluation in order to verify the operating temperature of the heating block.

To this end, the cell thawing machine 200 according to one embodiment of the present invention may further include a sensor insertion hole 180, and the sensor insertion hole 180 may be formed to be drawn at a certain depth from one surface of the heating blocks 122a, 122b.

The sensor insertion hole 180 may be a space into which a calibration sensor is inserted during calibration in order to check whether the heating blocks 122a, 122b are normally operating.

For example, the calibration sensor (not illustrated) may be a bar-shaped temperature sensor having a predetermined length, and a partial length of the calibration sensor may be inserted into the sensor insertion hole 180.

Accordingly, since the checker may easily measure the temperatures of the heating blocks 122a, 122b through the calibration sensor inserted into the sensor insertion hole 180, it is possible to check whether the cell thawing machines 100, 200 according to one embodiment of the present invention are normally operating.

Although the sensor insertion hole 180 may be provided as one, when the heating blocks 122a, 122b include the above-described first heating block 122a and second heating block 122b, it may be provided in plurality so as to be respectively formed in the first heating block 122a and the second heating block 122b.

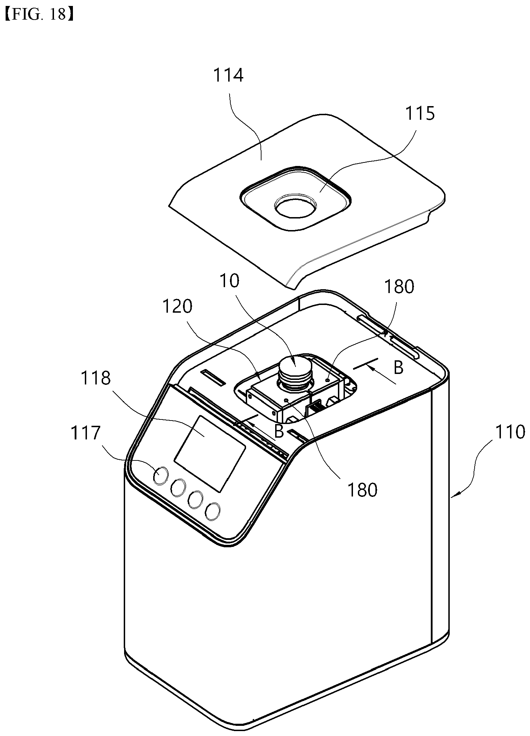

In this case, the sensor insertion hole 180 may be formed on a side surface of the heating blocks 122a, 122b, but as illustrated in FIG. 18, the sensor insertion hole 180 may be formed on the heating blocks 122a, 122b to be drawn in at a certain depth from the upper surface to the lower portion of the heating block 122a, 122b.

In addition, when the cell thawing machines 100, 200 according to one embodiment of the present invention include auxiliary heat insulating blocks 124c, 124d for covering the upper surfaces of the heating blocks 122a, 122b, the sensor insertion hole 180 may be formed on the heating blocks 122a, 122b so as to be inserted from the upper surface to the lower portion of the heating blocks 122a, 122b to a certain depth while penetrating the auxiliary heat insulating blocks 124c, 124d.

Accordingly, when the upper cover 114 on which the inlet 115 is formed is separated from the housing 110, the sensor insertion holes 180 formed on the upper surfaces of the heating blocks 122a, 122b and auxiliary heat insulating blocks 124c, 124d may be exposed to the outside.

As a result, even if only the upper cover 114 is separated from the housing 110 without the need to separate the entire housing in the cell thawing machines 100, 200 according to one embodiment of the present invention, the sensor insertion hole 180 for calibration may be exposed to the outside.

In addition, since the sensor insertion hole 180 is drawn at a certain depth from the upper surfaces to the lower portion of the heating block 122a, 122b in the cell thawing machines 100, 200 according to one embodiment of the present invention, the calibration sensor inserted into the sensor insertion hole 180 may maintain a state of being inserted upright into the sensor insertion hole 180 even without a separate fixing means.

Through this, after separating the upper cover 114, the checker may insert a partial length of the calibration sensor into the sensor insertion hole 180 formed on the upper surface of the heating block 122a, 122b, and then, it is possible to conveniently and accurately measure the operating temperatures of the heating block 122a, 122b.

In this case, the sensor insertion hole 180 may be formed to be located directly above the temperature sensors 128a, 128b that are embedded in the heating blocks 122a, 122b.

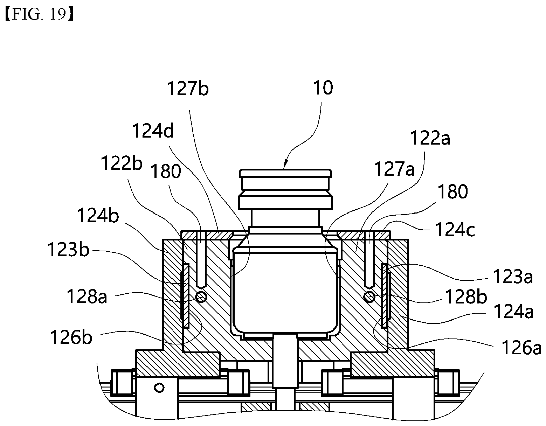

For example, as illustrated in FIG. 19, the sensor insertion hole 180 may be formed in the heating blocks 122a, 122b such that a lower end, which is a sealed end, is located directly above the temperature sensors 128a, 128b, and the lower end of the sensor insertion hole 180 may be formed on the heating block 122a, 122b to have a gap of 1 mm to 5 mm from the temperature sensor 128a, 128b.

Accordingly, during calibration, the temperatures of the heating blocks 122a, 122b measured by the calibration sensor inserted into the sensor insertion hole 180 may be similar to the temperatures of the heating blocks 122a, 122b measured by the temperature sensors 128a, 128b, when the cell thawing machines 100, 200 according to one embodiment of the present invention operate.

As a result, when calibration of the cell thawing machine 200 according to one embodiment of the present invention is performed by using the sensor insertion hole 180, the temperatures of the heating blocks 122a, 122b measured through the calibration sensor are similar to the temperatures of the heating blocks 122a, 122b measured by the temperature sensors 128a, 128b during normal operation, and thus, the calibration result may secure high reliability.

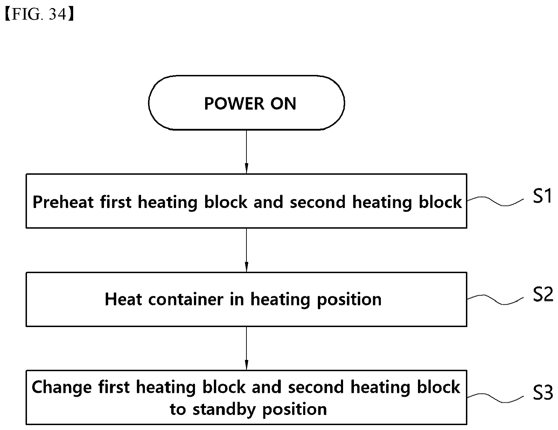

Hereinafter, the method for operating the above-described cell thawing machines 100, 200 will be described with reference to FIGS. 13 to 17 and FIGS. 29 to 34.

First of all, as a preparation step, a cover 116 for covering the inlet 115 is removed, and power is supplied to the cell thawing machines 100, 200.

When power is applied to the cell thawing machines 100, 200, the control part 150 may drive the driving part 130 to change the thawing part 120 to a standby position.

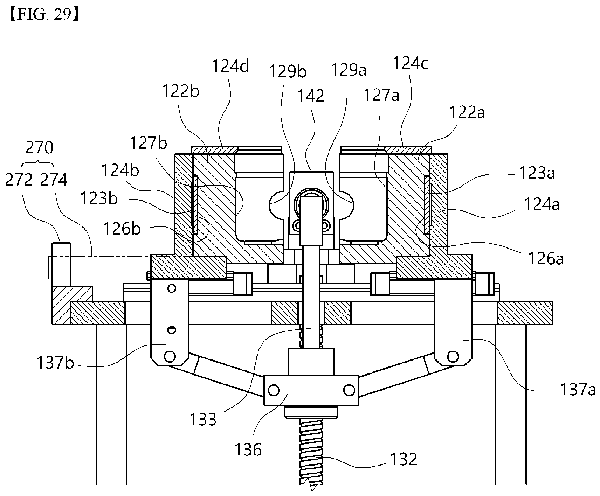

That is, as illustrated in FIGS. 13 and 29, the control part 150 may drive the motor 131 to raise the moving member 136. Accordingly, the first heating block 122a and the second heating block 122b may be slid and moved away from each other, and may be changed to a standby position of being spaced apart from each other at a predetermined interval.

If the first heating block 122a and the second heating block 122b are located in the standby position, the preparation step of changing the thawing part 120 to the standby position when power is applied may be omitted.

Next, as a first step (S1), the control part 150 may operate the first heater 123a and the second heater 123b to preheat the first heating block 122a and the second heating block 122b to a predetermined temperature.

For example, the preheating temperature of the first heating block 122a and the second heating block 122b may be equal to or relatively higher than the thawing temperature for thawing the biological material contained in the container 10.

In this case, the preheating of the first heating block 122a and the second heating block 122b may be performed in a state where surfaces of the first heating block 122a and the second heating block 122b facing each other are in contact with each other.

That is, as illustrated in FIGS. 14 and 30, the control part 150 may lower the moving member 136 by driving the motor 131. Accordingly, the first heating block 122a and the second heating block 122b may slid and moved in a direction closer to each other in the standby position, respectively, and they may be changed to a state where surfaces of the first heating block 122a and the second heating block 122b facing each other are in contact with each other.

In this state, the control part 150 may control the driving of the motor 131 such that the contact state of the first heating block 122a and the second heating block 122b may be maintained until the preheating of the first heating block 122a and the second heating block 122b is finished.

Accordingly, while the first heating block 122a and the second heating block 122b are in contact with each other, the first heating block 122a and the second heating block 122b may be simultaneously heated through a first heater 123a and a second heater 123b that are respectively placed on both sides, and thus, the first heating block 122a and the second heating block 122b may be quickly heated to a predetermined temperature through heat transferred from the first heater 123a and the second heater 123b.

Thereafter, when the preheating of the first heating block 122a and the second heating block 122b is completed, the control part 150 may output a message indicating that the preheating is completed to the display 118, and the control part 150 may slide and move the first heating block 122a and the second heating block 122b by driving the motor 131.

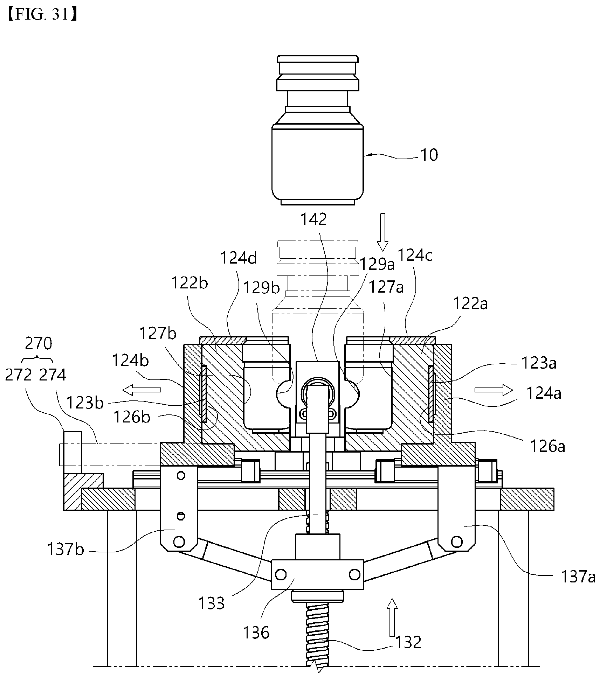

Through this, as illustrated in FIGS. 15 and 31, the first heating block 122a and the second heating block 122b may be changed to a standby position of being spaced apart from each other at a predetermined interval, and the support pins 133 may be raised upward to a predetermined height.

In this way, when the preheating of the first heating block 122a and the second heating block 122b is completed, the user may check the set thawing time through the display 118, and if the set thawing time needs to be changed, the user may manipulate the manipulation part 117 to change the set thawing time to an appropriate time.

Next, as illustrated in FIGS. 15 and 31, in a state where the first heating block 122a and the second heating block 122b are located in the standby position, the user may dispose a container 10 that needs to be thawed at the end of the support pins 133 that protrude upward at a certain height between the first heating block 122a and the second heating block 122b that are disposed in the standby position.

Thereafter, the user may input a thawing start signal through the manipulation part 117 in a state where the container 10 that needs to be thawed is disposed at the end of the support pins 133.

Accordingly, the control part 150 may heat the container 10 so as to thaw the biological material contained in the container 10 as a second step (S2).

That is, the control part 150 may lower the moving member 136 by driving the motor 131 such that the first heating block 122a and the second heating block 122b can be moved in a direction closer to each other.

Accordingly, as illustrated in FIGS. 16 and 31, the first heating block 122a and the second heating block 122b may be changed from a standby position to a heating position for forming a heating space 121 for accommodating and heating the container 10, and as described above, the support pins 133 may be lowered together with the moving member 136 such that one end protrudes from a portion forming the bottom surface of the heating space 121 at a certain height.

Accordingly, the container 10 may have a circumferential surface surrounded by the first heating block 122a and the second heating block 122b in a state of being disposed in the heating space 121, and a lower portion of the container 10 disposed in the heating space 121 may be disposed in the heating space 121 while being spaced apart from the bottom surface of the heating space 121 by a predetermined interval through the support pins 133, and the sensor 141 may measure the surface temperature of the container 10 in a very close position without contacting the container 10 that is disposed in the heating space 121 through the placement holes 129a, 129b.

Through this, the container 10 may be heated by the thawing time set by the user through the heat transferred from the first heating block 122a and the second heating block 122b, and through the sensor 141, it is possible to measure the state of the container 10 in real time.

In this case, the control part 150 may output a message indicating that it is thawing to the display 118, and based on the temperature information transmitted from the first temperature sensor 128a and the second temperature sensor 128b, the temperatures of the first heating block 122a and the second heating block 122b may be maintained to be constant by controlling the operation of the first heater 123a and the second heater 123b, and the state information about the container 10 detected through the sensor 141 may be output to the display 118 through driving of the control part 150.

In this case, as described above, while the container 10 disposed in the heating space 121 is not in contact with the first heating block 122a and the second heating block 122b, it may be heated by the heat transferred from the first heating block 122a and the second heating block 122b in a non-contact manner.

That is, while the container 10 is inserted into the heating space 121, the circumferential surface of the container 10 may be spaced apart by a predetermined interval with the opposing surface 127a of the first heating block 122a and the opposing surface 127b of the second heating block 122b forming the heating space 121, and the lower surface of the container 10 may be spaced apart by a predetermined height from a portion forming the bottom surface of the heating space 121 through the support pins 133.

Through this, a gap (d) may be formed between the pair of opposing surfaces 127a, 127b and the outer surface of the container 10 in a state where the container 10 is disposed in the heating space 121.

Accordingly, the heat stored in the first heating block 122a and the second heating block 122b may be transferred to the relatively low-temperature container 10 through convection and/or radiation instead of conduction.

As a result, since the circumferential surface of the container 10 may be heated through convective heat and/or radiant heat, the circumferential surface of the container 10 may be uniformly heated by convective heat and/or radiant heat transferred from the heating block 122a, 122b.

Additionally, in the second step (S2), the control part 150 may control the driving of the motor 131 so as to heat the container 10 in a state where the first heating block 122a and the second heating block 122b are in contact with each other, but it may control the driving of the motor 131 so as to heat the container 10 in a state where the first heating block 122a and the second heating block 122b do not contact each other.

That is, the control part 150 may set the heating position such that a predetermined gap is formed in the remaining portion except for portions where the opposing surfaces 127a, 127b for forming the heating space 121 are formed among the surfaces of the first heating block 122a and the second heating block 122b facing each other.

Through this, the cell thawing machines 100, 200 according to one embodiment of the present invention may prevent a load that may occur in the motor in order to maintain the state where the first heating block 122a and the second heating block 122b are in contact with each other in the heating position, and in the process of thawing the biological material contained in the container 10 at the heating position, it is possible to discharge water vapor or moisture generated from the surface of the container 10 to the outside through the gap, and thus, it is possible to thaw the biological material more smoothly.

Next, when the thawing time set by the user elapses, the control part 150 may output a message indicating completion to the display 118, and as a third step (S3), the control part 150 may drive the motor 131 to change the first heating block 122a and the second heating block 122b to a standby position.

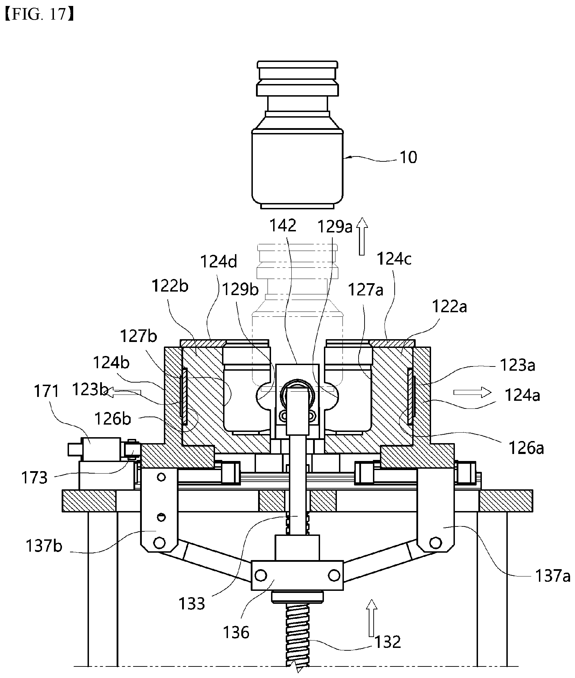

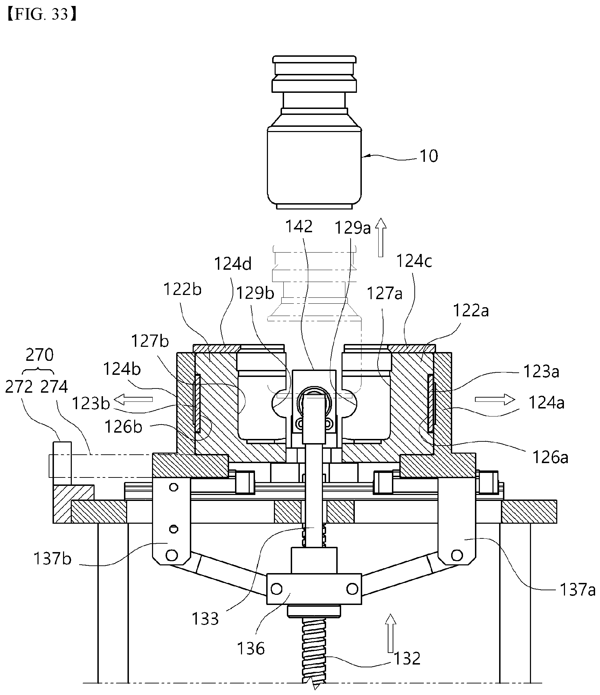

Accordingly, as illustrated in FIGS. 17 and 33, the user may remove the container 10 disposed between the first heating block 122a and the second heating block 122b while the first heating block 122a and the second heating block 122a are located in the standby position.

In this case, the control part 150 may control the driving of the first heater 123a and the second heater 123b such that the temperatures of the first heating block 122a and the second heating block 122b are continuously maintained to be constant.

Accordingly, the user may sequentially thaw a plurality of containers 10 by sequentially repeating the first step (S 1) and the second step (S2).

Although one exemplary embodiment of the present invention has been described above, the spirit of the present invention is not limited to the exemplary embodiments presented herein, and those skilled in the art who understand the spirit of the present invention may easily suggest other exemplary embodiments by changing, modifying, deleting or adding components within the scope of the same spirit, but this will also fall within the scope of the present invention.

## Claims

1. A cell thawing machine, comprising:
a thawing part comprising heating blocks which comprise a first heating block and a second heating block forming a heating space for heating a container in which a predetermined amount of biological material comprising cells is stored, heaters which are installed in the heating blocks to provide heat to the heating blocks, and heat insulating blocks which are coupled to the heating blocks so as to surround the heaters;
a driving part for providing a driving force for raising and lowering support pins such that the support pins can support a lower portion of the container, and linearly moving the first heating block and the second heating block to the left and right directions; and
a control part for controlling the thawing part and the driving part.

2. The cell thawing machine of claim 1, wherein the container disposed in the heating space is heated by heat transferred from the heating blocks in a non-contact manner in a state where a lower portion of the container is supported through the support pins and the container is not in contact with the heating blocks.

3. The cell thawing machine of claim 1, wherein the heating block is made of a thermally conductive material.

4. The cell thawing machine of claim 1, wherein the heating block comprises an arrangement groove which is formed to be drawn inward on one surface, the heater is inserted into the arrangement groove, and the heat insulating block is coupled to the heating block so to cover the arrangement groove.

5. The cell thawing machine of claim 1, wherein the heating space is formed through opposing surfaces that are each drawn inward to have a shape corresponding to the circumferential surface of the container on surfaces facing each other in the first heating block and the second heating block.

6. The cell thawing machine of claim 5, wherein the opposing surfaces are formed to surround a side portion and lower portion of the container.

7. The cell thawing machine of claim 1, wherein the heating block comprises a through-hole which is formed to penetrate such that an end of the support pin can pass toward the heating space.

8. The cell thawing machine of claim 1, wherein the thawing part further comprises an auxiliary heat insulating block which covers an upper surface of the heating block.

9. The cell thawing machine of claim 1, wherein the thawing part comprises a temperature sensor which is installed on the heating block to measure the temperature of the heating block.

10. The cell thawing machine of claim 9, wherein the cell thawing machine further comprises a sensor insertion hole which is formed to be drawn at a certain depth from an upper surface of the heating block to a lower portion such that a calibration sensor for measuring the temperature of the heating block can be inserted therein, and
wherein the sensor insertion hole is formed to be located directly above the temperature sensor.

11. The cell thawing machine of claim 1, wherein the driving part comprises a motor for providing a driving force, a rotating member which is axially coupled to a rotating shaft of the motor, a moving member which is raised and lowered along the rotating member when the rotating member rotates, the support pins which are coupled to the moving member such that the support pins can support a lower portion of the container by moving up and down along with the moving member, and a power transmission member for interconnecting the moving member and the thawing part such that the first heating block and the second heating block can be moved linearly in the left and right directions, respectively, through a driving force of the motor.

12. The cell thawing machine of claim 11, wherein the power transmission member comprises a guide block which is fixedly coupled to the heat insulating block, a guide rail for guiding the linear movement of the guide block, and a link member for linking the guide block and the moving member to each other.

13. The cell thawing machine of claim 1, wherein the cell thawing machine further comprises a sensor for detecting the state of the container disposed in the heating space.

14. The cell thawing machine of claim 1, wherein the cell thawing machine further comprises a limit switch for detecting the position of the heating block through contact with the heat insulating block.

15. The cell thawing machine of claim 1, wherein the cell thawing machine further comprises a non-contact position detection sensor for detecting the position of the heating block.

16. The cell thawing machine of claim 1, wherein the cell thawing machine comprises a housing having an inlet which is formed through a position corresponding to the heating space.

17. The cell thawing machine of claim 16, wherein the cell thawing machine further comprises a sensor insertion hole which is formed to be drawn at a certain depth from an upper surface of the heating block to a lower portion such that a calibration sensor for measuring the temperature of the heating block can be inserted therein,
wherein the housing comprises a box-shaped main body and an upper cover which is detachably coupled to the main body so as to cover an upper portion of the main body,
wherein the inlet is formed on the upper cover to be located at a position corresponding to the heating space, and
wherein the sensor insertion hole is exposed to the outside when the upper cover and the main body are separated.

18. The cell thawing machine of claim 1, wherein when heating the container accommodated in the heating space, the control part controls the driving of the driving part to heat the container in a state where the first heating block and the second heating block are not in contact with each other.

19. The cell thawing machine of claim 1, wherein the control part individually controls the temperatures of the first heating block and the second heating block.

20. A method for operating a cell thawing machine comprising heating blocks which comprise a first heating block and a second heating block for forming a heating space for accommodating a container in which a predetermined amount of biological material comprising cells is stored through linear movement by a driving force of a motor, and heating the container accommodated in the heating space through heat provided from a heater; and support pins which are raised and lowered to support a lower portion of the container disposed in the heating space in connection with the linear movement of the heating block, the method comprising:
a first step of preheating the first heating block and the second heating block to a predetermined temperature by driving the heater while one surface of the first heating block and one surface of the second heating block facing each other are in contact with each other;
a second step of heating the container disposed in the heating space to thaw the biological material while the first heating block and the second heating block are changed to a heating position forming the heating space; and
a third step of changing the first heating block and the second heating block to a standby position by moving the same in a direction away from the heating position such that the container disposed in the heating space can be removed,
wherein in the second step, the container disposed in the heating space is heated by heat transferred from the first heating block and the second heating block in a non-contact manner in a state where a lower portion of the container is supported through the support pins and the container is not in contact with the first heating block and the second heating block.
